(19) Europäisches Patentamt
European Patent Office
Office européen des brevets

(11) Veröffentlichungsnummer: **0 139 182**
**A2**

(12) # EUROPÄISCHE PATENTANMELDUNG

(21) Anmeldenummer: **84110329.4**

(22) Anmeldetag: **30.08.84**

(51) Int. Cl.⁴: **A 01 N 43/56**
A 01 N 47/16, A 01 N 47/18
A 01 N 47/36, A 01 N 47/38
A 01 N 57/32, C 07 D 231/38
C 07 D 231/40, C 07 D 231/16
C 07 F 9/65

(30) Priorität: **07.09.83 DE 3332270**

(43) Veröffentlichungstag der Anmeldung:
**02.05.85 Patentblatt 85/18**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Maurer, Fritz, Dr.**
**Roeber Strasse 8**
**D-5600 Wuppertal 1(DE)**

(72) Erfinder: **Lunkenheimer, Winfried, Dr.**
**Bismarckstrasse 29**
**D-5600 Wuppertal 1(DE)**

(72) Erfinder: **Fedtke, Carl, Dr.**
**Wehrheiderstrasse 5**
**D-5000 Köln 80(DE)**

(54) **Herbizide Mittel enthaltend Photosynthesehemmer-Herbizide in Kombination mit 1,4-disubstituierten Pyrazol-Derivaten.**

(57) Die neuen synergistischen Wirkstoffkombinationen bestehend aus (1) einem Photosynthesehemmer-Wirkstoff (Herbizid) und (2) einem 1,4-disubstituierten Pyrazol-Derivat der allgemeinen Formel (II) (Synergist),

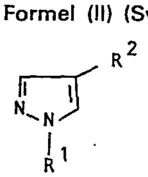

(II)

(worin $R^1$ und $R^2$ die in der Beschreibung angebenen Bedeutung haben), weisen eine besonders hohe herbizide Wirksamkeit auf.

Ein besonders charakteristisches Beispiel für die Photosynthesehemmer-Wirkstoffe ist 4-Amino-6-tert.-butyl-3-methylthio-1,2,4-triazin-5-on (Metribuzin).

EP 0 139 182 A2

BAYER AKTIENGESELLSCHAFT
Konzernverwaltung RP
Patentabteilung

5090 Leverkusen, Bayerwerk

Bi/Th
III

# Herbizide Mittel enthaltend Photosynthesehemmer-Herbizide in Kombination mit 1,4-disubstituierten Pyrazol-Derivaten

Die vorliegende Erfindung betrifft neue herbizide synergistische Wirkstoffkombinationen, die aus bekannten Photosyntesehemmer-Herbiziden einerseits und aus neuen 1,4-disubstituierten Pyrazol-Derivaten andererseits besteht.

Es ist bereits bekannt geworden, daß bestimmte Herbizide, wie z.B. 4-Amino-6-tert.-butyl-3-methylthio-1,2,4-triazin-5-on, photosynthese-hemmende Eigenschaften besitzen (vergl.z.B. Carl Fedtke, Biochemistry and Physiology of Herbicide Action, Springer Verlag, 1982). Nachteilig bei dieser herbiziden Verbindung ist jedoch, daß nicht immer alle vorkommenden Unkräuter und Ungräser voll erfaßt werden oder aber, daß bei entsprechend hohen . Aufwandmengen einige Kulturpflanzenarten teilweise geschädigt werden.

Es wurde gefunden, daß die neuen Wirkstoffkombinationen, bestehend aus

a) einem Photosynthesehemmer-Wirkstoff (Herbizid) und

b) einem 1,4-disubstituierten Pyrazol-Derivat der allgemeinen Formel (II) (Synergist),

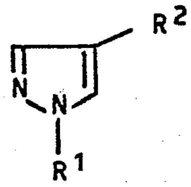 (II)

in welcher

$R^1$ für Alkyl mit mehr als zwei Kohlenstoffatomen, gegebenenfalls substituiertes Cycloalkyl, substituiertes Benzyl, sowie die Gruppierungen

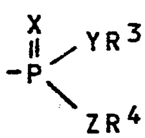 ; $-CO-XR^5$ und 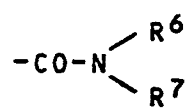 steht,

$R^2$ für Amino, Alkylamino sowie die Gruppierungen

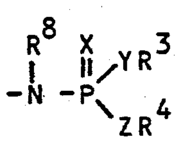 , 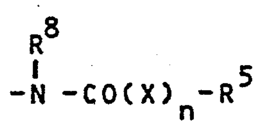 und 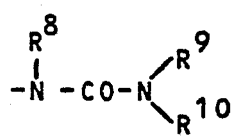 steht,

sowie auch für Nitro steht, wenn $R^1$ nicht für substituiertes Benzyl steht;

$R^3$ für Alkyl steht;

$R^4$ für Alkyl steht;

$R^5$ für Alkyl oder Halogenalkyl steht;

$R^6$ für Wasserstoff oder Alkyl steht;

$R^7$ für Alkyl, Halogenalkylthio, Cyanalkylthio oder gegebenenfalls substituiertes Phenyl steht, wobei jedoch $R^6$ und $R^7$ nicht gleichzeitig für Methyl stehen;

$R^8$ für Wasserstoff oder Alkyl steht;

$R^9$ für Wasserstoff oder Alkyl steht;

$R^{10}$ für Alkyl, Halogenalkylthio, Cyanalkylthio oder gegebenenfalls substituiertes Phenyl steht;

$R^9$ und $R^{10}$ gemeinsam mit dem Stickstoffatom, an das sie gebunden sind, für einen Heterocyclus stehen, der weitere Heteroatome enthalten kann;

X, Y und Z für Sauerstoff oder Schwefel stehen und der Index

n für 0 oder 1 steht,

eine besonders hohe herbizide Wirksamkeit aufweisen.

Ueberraschenderweise ist die herbizide Wirksamkeit der erfindungsgemäßen Wirkstoffkombinationen wesentlich höher als die Summe der Wirkungen der einzelnen Wirkstoffe. Insbesondere besitzen eine Vielzahl der neuen 1,4-disubstituierten Pyrazol-Derivate der allgemeinen Formel (II) bei den üblichen Aufwandmengen keine herbizide Wirkung, bewirken aber die Steigerung der herbiziden Wirkung der Photosynthesehemmer-Wirkstoffe. Somit ist der hier gefundene synergistische Effekt völlig unerwartet und überraschend.

Da der synergistische Effekt auch solche Unkräuter betrifft, die durch die verwendeten Photosynthesehemmer-Wirkstoffe bei alleiniger Ausbringung in üblichen Aufwandmengen nur unzureichend geschädigt oder gar nicht erfaßt werden, stellen die erfindungsgemäßen synergistischen Wirkstoffkombinationen eine wertvolle Bereicherung der Technik dar.

Als Photosynthesehemmer-Wirkstoffe für die erfindungsgemäßen Wirkstoffkombinationen seien vorzugsweise die folgenden der allgemeinen Formeln (I-A) bis (I-J) genannt:

(A) Triazinon-Derivate der Formel

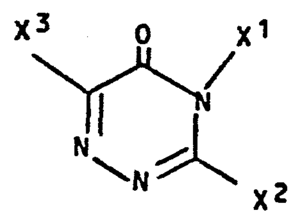

(I-A)

in welcher

$X^1$ für Amino, gegebenenfalls substituiertes Alkylidenamino oder Alkyl mit 1 bis 2 Kohlenstoffatomen steht;

$X^2$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^3$ für gegebenenfalls durch Halogen substituiertes tert.-Butyl oder gegebenenfalls substituiertes Phenyl steht;

(B) Triazindion-Derivate der Formel

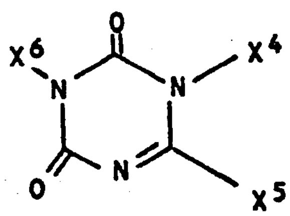

(I-B)

in welcher

$X^4$ für Amino, gegebenenfalls substituiertes Alkylidenamino oder Alkyl mit 1 bis 2 Kohlenstoffatomen steht;

$X^5$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^6$ für Alkyl mit 1 bis 6 Kohlenstoffatomen oder gegebenenfalls substituiertes Phenyl steht;

(C) Triazin-Derivate der Formel

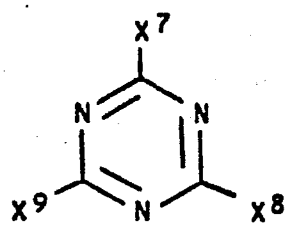

(I-C)

in welcher

$X^7$ für Chlor, Alkoxy oder Alkylthio mit jeweils 1 bis 2 Kohlenstoffatomen steht;

$X^8$ für Alkylamino mit 1 bis 4 Kohlenstoffatomen im Alkylteil steht; und

$X^9$ für gegebenenfalls durch Cyano substituiertes Alkyl mit 1 bis 4 Kohlenstoffatomen steht;

(D) Harnstoff-Derivate der Formel

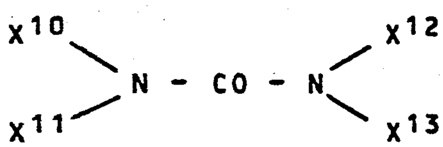

(I-D)

in welcher

$X^{10}$ für gegebenenfalls substituiertes Phenyl, Benzthiazolyl oder gegebenenfalls substituiertes Thiadiazolyl steht;

$X^{11}$ für Wasserstoff oder Methyl steht;

$X^{12}$ für Methyl steht; und

$X^{13}$ für Wasserstoff, Alkyl mit 1 bis 4 Kohlenstoffatomen, Alkoxy mit 1 bis 2 Kohlenstoffatomen oder Alkinyl mit 2 bis 4 Kohlenstoffatomen steht;

(E) Carboxanilid-Derivate der Formel

$$X^{14} - CO - NH - X^{15} \quad \text{(I-E)}$$

in welcher

$X^{14}$ für Alkyl mit bis zu 6 Kohlenstoffatomen, Alkoxy mit 1 bis 4 Kohlenstoffatomen, Alkenyl mit 2 bis 4 Kohlenstoffatomen oder Cycloalkyl mit 3 bis 6 Kohlenstoffatomen steht; und

$X^{15}$ für gegebenenfalls substituiertes Phenyl steht;

(F) Uracil-Derivate der Formel

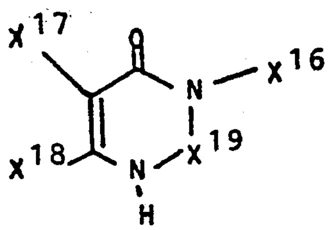

(I-F)

in welcher

$X^{16}$ für Alkyl mit 1 bis 6 Kohlenstoffatomen oder Cycloalkyl mit 5 bis 7 Kohlenstoffatomen steht;

$X^{17}$ für Halogen steht;

$X^{18}$ für Alkyl mit 1 bis 2 Kohlenstoffatomen steht, oder

$X^{17}$ und $X^{18}$ gemeinsam für eine gegebenenfalls substituierte Alkylenkette oder einen gegebenenfalls substituierten anellierten Benzolring stehen; und

$X^{19}$ für die -CO-oder $-SO_2$-Gruppe steht;

(G) Biscarbamat-Derivate der Formel

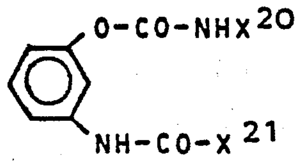

(I-G)

in welcher

$X^{20}$ für Alkyl mit 1 bis 4 Kohlenstoffatomen oder gegebenenfalls substituiertes Phenyl steht; und

$X^{21}$ für Alkoxy mit 1 bis 4 Kohlenstoffatomen oder Dialkylamino mit 1 bis 2 Kohlenstoffatomen in jedem Alkylteil steht;

(H) Pyridazinon-Derivate der Formel

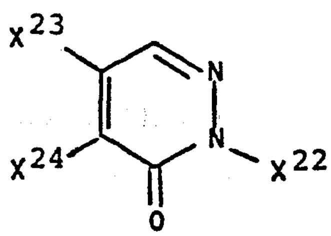

(I-H)

in welcher

$X^{22}$ für gegebenenfalls substituiertes Phenyl steht;

$X^{23}$ für Amino, Alkylamino oder Dialkylamino mit jeweils 1 oder 2 Kohlenstoffatomen in jedem Alkylteil steht; und

$X^{24}$ für Halogen steht;

(J) Hydroxybenzonitril-Derivate der Formel

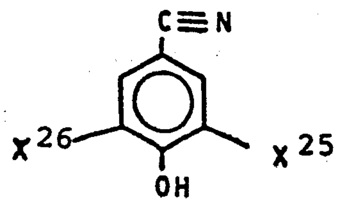

(I-J)

in welcher

$X^{25}$ für Halogen steht; und

$X^{26}$ für Halogen steht.

Besonders bevorzugt sind folgende Photosynthesehemmer-Wirkstoffe der allgemeinen Formeln (I-A) bis (I-J):

(A) Triazinon-Derivate der Formeln

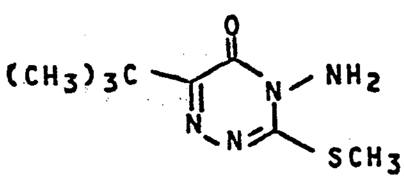

(I-A-1)
(Metribuzin)

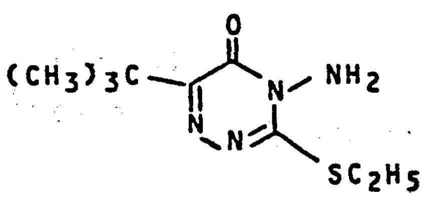

(I-A-2)

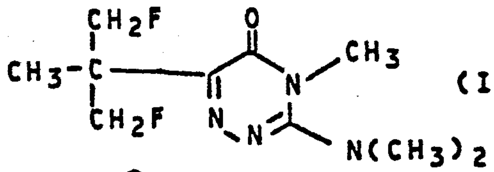

(I-A-3)

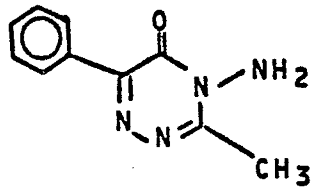

(I-A-4)
(Metamitron)

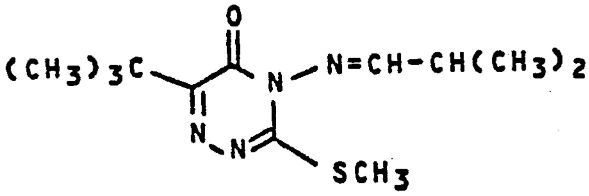

(I-A-5)
(Isomethiozin)

(B) Triazindion-Derivate der Formeln

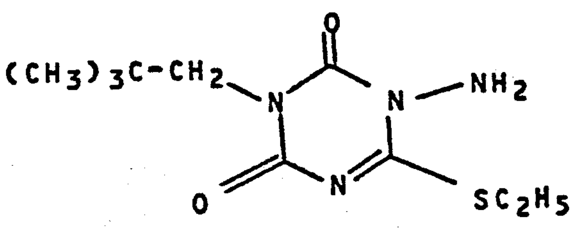

(I-B-1)
(Ametridione)

(C) Triazin-Derivate der Formeln

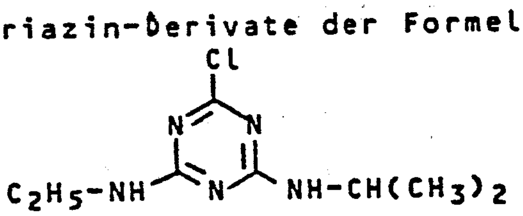

(I-C-1)
(Atrazine)

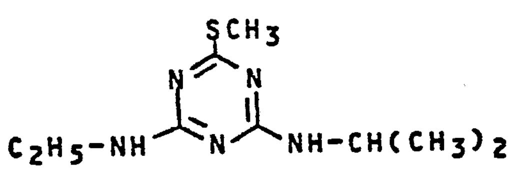

(I-C-2)
(Ametryne)

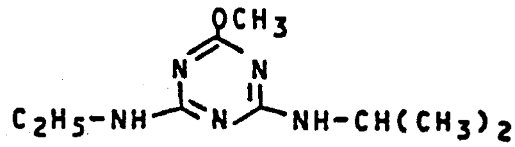

(I-C-3)
(Atraton)

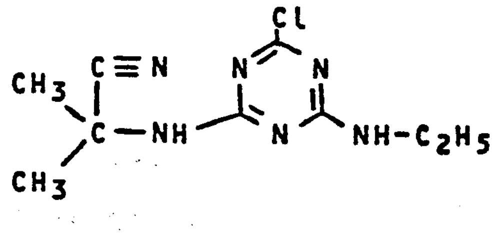

(I-C-4)
(Cyanazine)

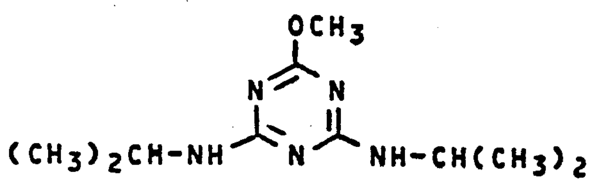

(I-C-5)
(Prometon)

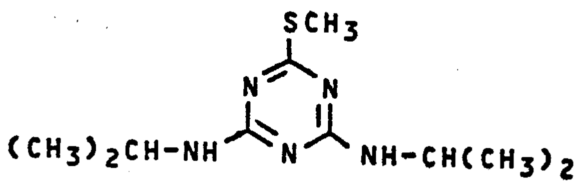

(I-C-6)
(Prometryne)

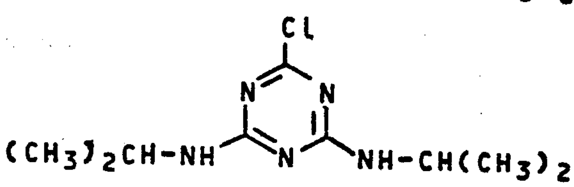

(I-C-7)
(Propazine)

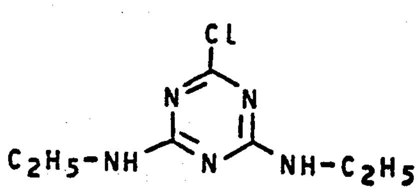

(I-C-8)
(Simazine)

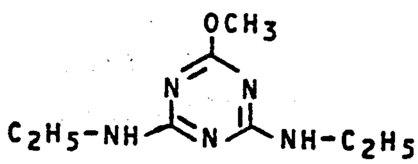

(I-C-9)
(Simeton)

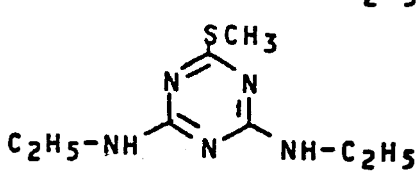

(I-C-10)
(Simetryne)

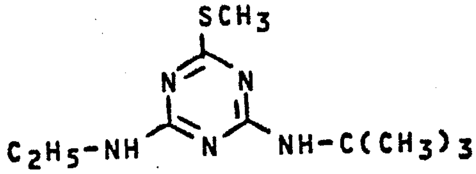

(I-C-11)
(Terbutryne)

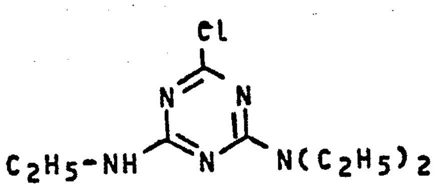

(I-C-12)
(Trietazine)

(D) Harnstoff-Derivate der Formeln

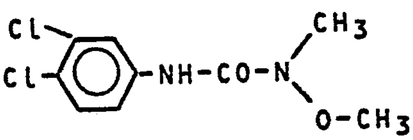

(I-D-1)
(Linuron)

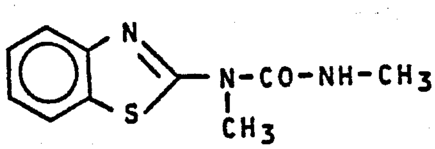

(I-D-2)
(Methabenzthiazuron)

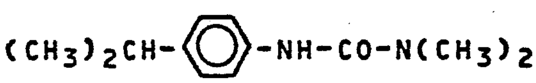

(I-D-3)
(Isoproturon)

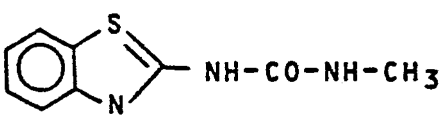

(I-D-4)
(Benzthiazuron)

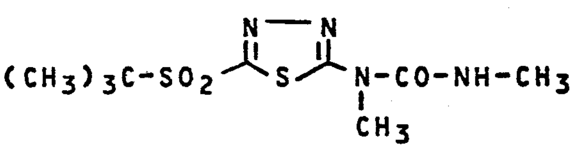

(I-D-5)
(Buthiuron)

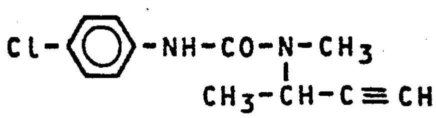

(I-D-6)
(Buturon)

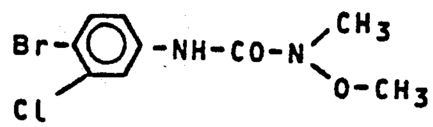

(I-D-7)
(Chlorbromuron)

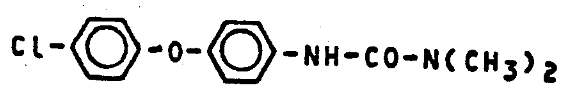

(I-D-8)
(Chloroxuron)

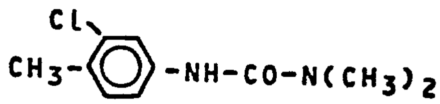

(I-D-9)
(Chlortoluron)

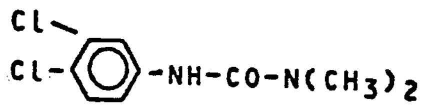

(I-D-10)
(Diuron)

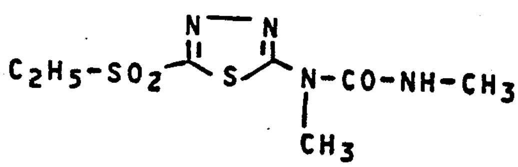

(I-D-11)
(Ethidimuron)

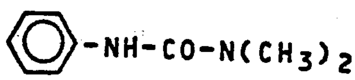

(I-D-12)
(Fenuron)

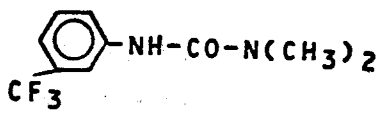

(I-D-13)
(Fluometuron)

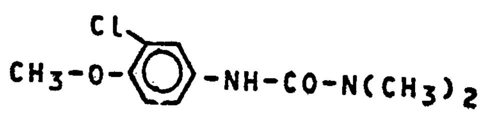

(I-D-14)
(Metoxuron)

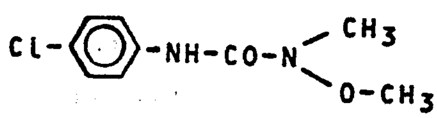

(I-D-15)
(Monolinuron)

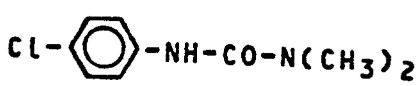

(I-D-16)
(Monuron)

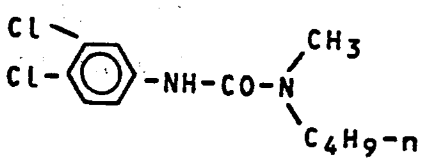

(I-D-17)
(Neburon)

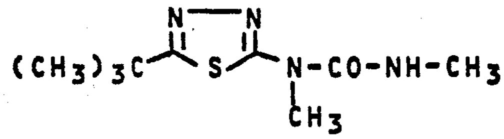

(I-D-18)
(Tebuthiuron)

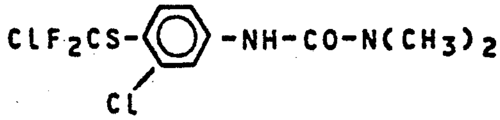

(I-D-19)
(Thiochlormethyl)

(E) Carboxanilid-Derivate der Formeln

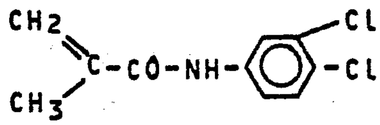

(I-E-1)
(Chloranocryl)

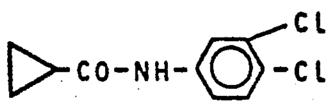

(I-E-2)
(Cypromid)

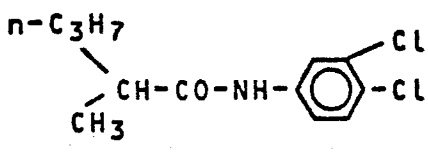

(I-E-3)
(Karsil)

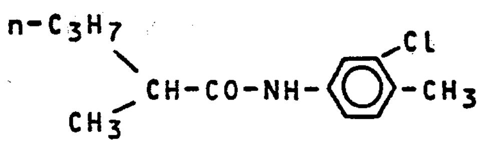

(I-E-4)
(Pentanochlor)

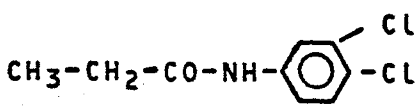

(I-E-5)
(Propanil)

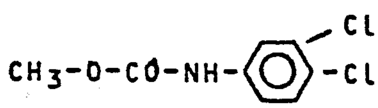

(I-E-6)
(Swep)

(F) Uracil-Derivate der Formeln

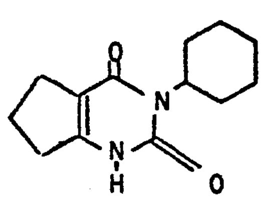

(I-F-1)
(Lenacil)

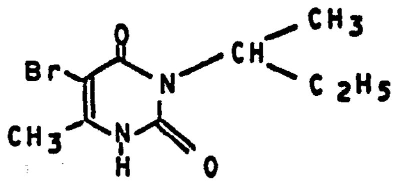

(I-F-2)
(Bromacil)

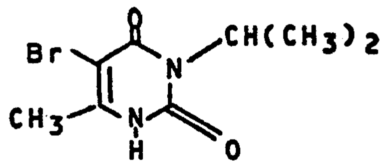

(I-F-3)
(Isocil)

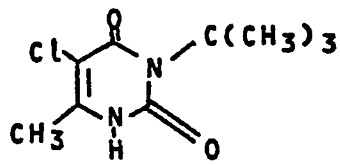

(I-F-4)
(Terbacil)

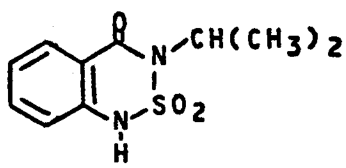

(I-F-5)
(Bentazon)

(G) Biscarbamat-Derivate der Formeln

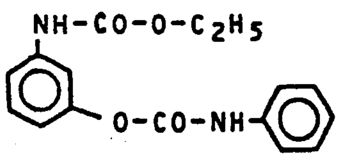

(I-G-1)
(Desmedipham)

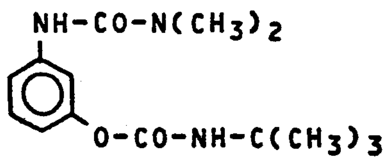

(I-G-2)
(Karbutilate)

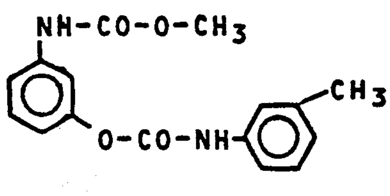

(I-G-3)
(Phenmedipham)

0139182

(H) Pyridazinon-Derivate der Formeln

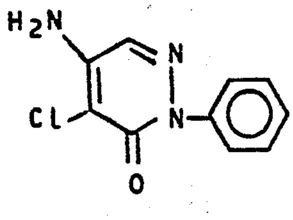

(I-H-1)
(Pyrazone)

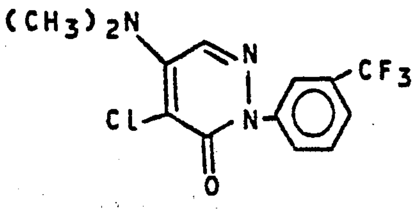

(I-H-2)
(Metflurazone)

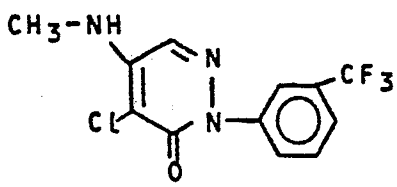

(I-H-3)
(Norflurazon)

(J) Hydroxybenzonitril-Derivate der Formeln

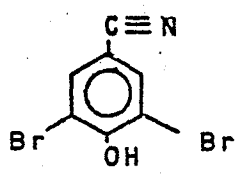

(I -J-1)
(Bromoxynil)

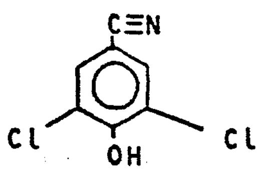

(I -J-2)
(Chloroxynil)

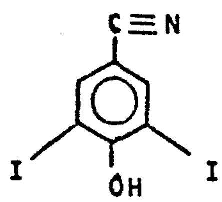

(I -J-3)
(Ioxynil)

Die Photosynthesehemmer-Wirkstoffe der Formeln (I-A)bis I-J) sind bekannt (vergl. z.B. Carl Fedtke, Biochemistry and Physiology of Herbicide Action, Springer-Verlag, 1982).

Die weiterhin als Mischungskomponente zu verwendenden 1,4-disubstituierten Pyrazol-Derivate sind durch die Formel (II) allgemein definiert. In dieser Formel stehen vorzugsweise

$R^1$ für Alkyl mit 3 bis 6 Kohlenstoffatomen, für gegebenenfalls durch Alkyl mit 1 bis 2 Kohlenstoffatomen substituiertes Cycloalkyl mit 5 oder 6 Kohlenstoffatomen, für substituiertes Benzyl, wobei als Substituenten genannt seien : Halogen, Alkyl mit 1 bis 2 Kohlenstoffatomen, Halogenalkyl mit 1 bis 2 Kohlenstoffatomen und 1 bis 5 gleichen oder verschiedenen Halogenatomen, wie Fluor- und Chloratomen; sowie für die Gruppierungen

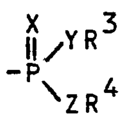 , $-CO-XR^5$ und $-CO-NR^6R^7$ ;

$R^2$ für Amino, für Alkylamino mit 1 bis 4 Kohlenstoffatomen im Alkylteil, für die Gruppierungen

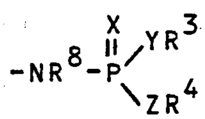 , $-NR^8-CO-(X)_n-R^5$ und $-NR^8-CO-NR^9R^{10}$ sowie auch für Nitro, wenn $R^1$ nicht für substituiertes Benzyl steht;

$R^3$ für Alkyl mit 1 bis 4 Kohlenstoffatomen;

$R^4$ für Alkyl mit 1 bis 4 Kohlenstoffatomen;

$R^5$ für Alkyl mit 1 bis 4 Kohlenstoffatomen oder für Halogenalkyl mit 1 bis 2 Kohlenstoffatomen und 1 bis 5 gleichen oder verschiedenen Halogenatomen, wie Fluor- und Chloratomen;

$R^6$ für Wasserstoff oder Alkyl mit 1 bis 4 Kohlenstoffatomen;

$R^7$ für Alkyl mit 1 bis 4 Kohlenstoffatomen, für Halogenalkylthio mit 1 bis 2 Kohlenstoffatomen und 1 bis 5 gleichen oder verschiedenen Halogenatomen, wie Fluor- und Chloratomen, für Cyanalkylthio mit 1 bis 4 Kohlenstoffatomen im Alkylteil, sowie für gegebenenfalls substituiertes Phenyl, wobei als Substituenten die bei $R^1$ genannten Benzylsubstituenten infrage kommen; wobei jedoch $R^6$ und $R^7$ nicht gleichzeitig für Methyl stehen;

$R^8$ für Wasserstoff oder Alkyl mit 1 bis 4 Kohlenstoffatomen steht;

$R^9$ für Wasserstoff oder Alkyl mit 1 bis 4 Kohlenstoffatomen steht;

$R^{10}$ für Alkyl mit 1 bis 4 Kohlenstoffatomen, für Halogenalkylthio mit 1 bis 2 Kohlenstoffatomen und 1 bis 5 gleichen oder verschiedenen Halogenatomen, wie Fluor- und Chloratomen, für Cyanalkylthio mit 1 bis 4 Kohlen-

stoffatomen im Alkylteil, sowie für gegebenenfalls substituiertes Phenyl, wobei als Substituenten die bei $R^1$ genannten Benzylsubstituenten infrage kommen;

$R^9$ und $R^{10}$ gemeinsam mit dem Stickstoffatom, an das sie gebunden sind, für einen fünf- oder sechsgliedrigen Heterocyclus stehen, der 1 bis 2 Stickstoffatome als mögliche Heteroatome enthalten kann;

X, Y und Z für Sauerstoff oder Schwefel und der Index

n für 0 oder 1.

Besonders bevorzugt sind Verbindungen der Formel (I), in denen

$R^1$ für geradkettiges oder verzweigtes Alkyl mit 3 bis 5 Kohlenstoffatomen, für gegebenenfalls durch Methyl substituiertes Cyclohexyl, für durch Fluor, Chlor, Methyl und/oder Trifluormethyl substituiertes Benzyl steht, sowie für die Gruppierungen

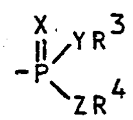 , $-CO-XR^5$ und 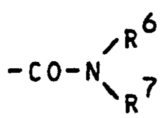 steht;

$R^2$ für Amino, Methylamino, Ethylamino, Propylamino oder die Gruppierungen

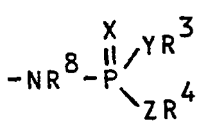 , $-NR^8-CO-(X)_n-R^5$ und $-NR^8-CO-NR^9R^{10}$ steht,

sowie auch für Nitro steht, wenn $R^1$ nicht für substituiertes Benzyl steht;

$R^3$ für Methyl, Ethyl oder Propyl steht;

$R^4$ für Methyl, Ethyl oder Propyl steht;

$R^5$ für Methyl, Ethyl, Trichlormethyl, Trifluormethyl, Trichlorethyl oder Trifluorethyl steht;

$R^6$ für Wasserstoff, Methyl oder Ethyl steht;

$R^7$ für Methyl, Ethyl, Halogenmethylthio mit 1 bis 3 gleichen oder verschiedenen Halogenatomen, wie Fluor- und Chloratomen, für Cyanalkylthio mit 1 bis 3 Kohlenstoffatomen im Alkylteil, sowie für gegebenenfalls durch Fluor, Chlor, Methyl und/oder Trifluormethyl substituiertes Phenyl steht, wobei jedoch nicht gleichzeitig $R^6$ und $R^7$ für Methyl stehen;

$R^8$ für Wasserstoff, Methyl, Ethyl, n-Propyl oder Isopropyl steht;

$R^9$ für Wasserstoff, Methyl oder Ethyl steht;

$R^{10}$ für Methyl, Ethyl, n-Propyl, Isopropyl, Halogenmethylthio mit 1 bis 3 gleichen oder verschiedenen Halogenatomen, wie Fluor- und Chloratomen, für Cyanalkylthio mit 1 bis 3 Kohlenstoffatomen im Alkylteil, sowie für gegebenenfalls durch Fluor, Chlor, Methyl und/oder Trifluormethyl substituiertes Phenyl steht;

$R^9$ und $R^{10}$ gemeinsam mit dem N-Atom, an das sie gebunden sind, für einen Pyrazol-Rest stehen;

X, Y und Z für Sauerstoff oder Schwefel stehen und der Index

n für 0 oder 1 steht.

Die 1,4-disubstituierten Pyrazol-Derivate der Formel (II) sind noch nicht bekannt. Sie können in allgemein bekannter und üblicher Weise erhalten werden, indem man

A) 1-(substituierte)Pyrazol-Derivate der Formel (III),

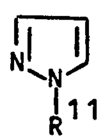

(III)

in welcher

$R^{11}$ für Wasserstoff, Alkyl mit mehr als zwei Kohlenstoffatomen, gegebenenfalls substituiertes Cycloalkyl oder substituiertes Benzyl steht,

in bekannter Weise mit Nitriersäure zu den 4-Nitropyrazolyl-Derivaten umsetzt;

und gegebenenfalls

B) die gemäß Verfahren (A) erhaltenen 4-Nitro-pyrazolyl-Derivate der Formel (IIa),

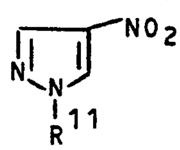

(IIa)

in welcher

$R^{11}$ die oben angegebene Bedeutung hat,

in bekannter Weise mit Wasserstoff in Gegenwart eines Katalysators und in Gegenwart eines polaren Verdünnungsmittels reduziert;

und gegebenenfalls

C) die gemäß Verfahren (A) oder (B) erhaltenen substituierten Pyrazolyl-Derivate der Formel (IIb),

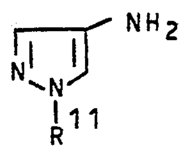

(IIb)

in welcher

$R^{11}$ die oben angegebene Bedeutung hat,

in üblicher Weise an der $NH_2$-Gruppe und/oder am N1-Atom für $R^{11}$ = Wasserstoff derivatisiert; und zwar

1.) durch Umsetzung mit Halogen-Derivaten der Formeln (IV), (V) und (VI),

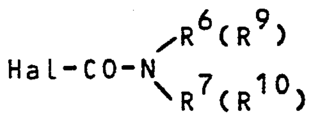

(IV)

in welcher

$R^6(R^9)$ und $R^7(R^{10})$ die oben angegebene Bedeutung haben und

Hal für Chlor oder Fluor steht ,

bzw.

$$Hal\text{-}CO\text{-}XR^5 \qquad (V)$$

in welcher

$R^5$ und Hal die oben angegebene Bedeutung haben,

bzw.

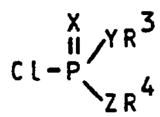

(VI)

in welcher

$R^3$, $R^4$, X, Y und Z die oben angegebene Bedeutung haben,

jeweils in Gegenwart eines Verdünnungsmittels und eines Säurebindemittels;

oder

2.) durch Umsetzung mit Isocyanaten der Formel (VII),

$$O{=}C{=}N\text{-}R^{12} \qquad (VII)$$

in welcher

$R^{12}$ für Alkyl oder gegebenenfalls substituiertes Phenyl steht;

in Gegenwart eines Verdünnungsmittels und gegebenenfalls in Gegenwart eines Katalysators;

oder

3.) durch Umsetzung mit Phosgen und anschließend mit Aminen der Formel (VIII),

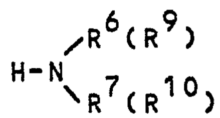

(VIII)

in welcher

$R^6(R^9)$ und $R^7(R^{10})$ die oben angegebene Bedeutung haben,

oder mit Alkoholen der Formel (IX),

$$H-O-R^5 \qquad (IX)$$

in welcher

$R^5$ die oben angegebene Bedeutung hat,

jeweils in Gegenwart eines Verdünnungsmittels und eines Säurebindemittels;

und gegebenenfalls

D) die gemäß Verfahren (C) erhaltenen substituierten Pyrazolyl-Derivate der Formel (IIc),

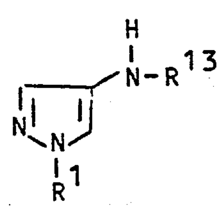

(IIc)

in welcher

$R^1$ die oben angegebene Bedeutung hat und

$R^{13}$ für die Gruppierungen

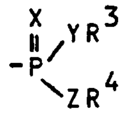 , $-CO-(X)_n-R^5$ und 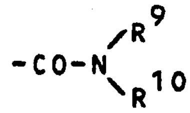 steht, wobei

$R^3$, $R^4$, $R^5$, $R^9$, $R^{10}$, X, Y und Z und der Index n die oben angegebene Bedeutung haben,

mit Alkylhalogeniden der Formel (X),

$$R^{14}\text{-Hal} \qquad (X)$$

in welcher

Hal für Brom und Iod steht und

$R^{14}$ für Alkyl mit 1 bis 4 Kohlenstoffatomen steht,

in Gegenwart einer Base und gegebenenfalls in Gegenwart eines Verdünnungsmittels umsetzt;

und gegebenenfalls

E) die gemäß Verfahren (D) erhaltenen substituierten Pyrazolyl-Derivate der Formel (IId),

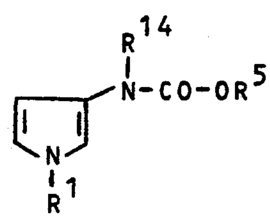 (IId)

in welcher
$R^1$, $R^5$ und $R^{14}$ die oben angegebene Bedeutung haben,

in Gegenwart eines Verdünnungsmittels mit wäßrig-alkalischer Lösung behandelt;

und gegebenenfalls

F) die gemäß Verfahren (E) erhaltenen substituierten Pyrazolyl-Derivate der Formel (IIe)

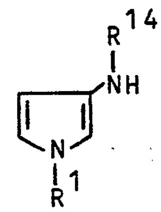

(IIe)

in welcher
$R^1$ und $R^{14}$ die oben angegebene Bedeutung haben,

gemäß Verfahren (C) umsetzt.

Die als Ausgangsstoffe zu verwendenden Pyrazol-Derivate sind durch die Formel (III) allgemein definiert. In dieser Formel steht $R^{11}$ vorzugsweise für Wasserstoff, für Alkyl mit 3 bis 6 Kohlenstoffatomen, für gegebenenfalls durch Alkyl mit 1 bis 2 Kohlenstoffatomen substituiertes Cycloalkyl mit 5 oder 6 Kohlenstoffatomen, sowie für gegebenenfalls durch Halogen, Alkyl mit 1 bis 2 Kohlenstoffatomen und/oder Halogenalkyl mit 1 bis 2 Kohlenstoffatomen und 1 bis 5 gleichen oder verschiedenen Halogenatomen, wie Fluor- und Chloratomen, substituiertes Benzyl.

Die Pyrazol-Derivate der Formel (III) sind bekannt bzw. können sie in allgemein üblicher Art und Weise erhalten werden, indem man entsprechend substituierte Hydrazine, vorzugsweise in Form ihrer Hydrochloride, in Gegenwart eines Verdünnungsmittels und in Gegenwart einer starken Säure, wie z.B. Salzsäure, mit 1,1,3,3-Tetramethoxypropan umsetzt (vgl. auch die Herstellungsbeispiele).

Die außerdem als Ausgangsstoffe zu verwendenden Halogen-Derivate sind durch die Formeln (IV), (V) und (VI) allallgemein definiert. In diesen Formeln stehen $R^3$, $R^4$, $R^5$, $R^6$, $R^7$, $R^9$, $R^{10}$, X, Y und Z vorzugsweise für die Reste, die bei den Verbindungen der Formel (II) bereits vorzugsweise für diese Substituenten genannt werden.

Die Halogen-Derivate der Formeln (IV), (V) und (VI) sind allgemein bekannte Verbindungen der organischen Chemie.

Die weiterhin als Ausgangsstoffe zu verwendenden Isocyanate sind durch die Formel (VII) allgemein definiert. In dieser Formel steht $R^{12}$ vorzugsweise für Alkyl mit 1 bis 4 Kohlenstoffatomen sowie für gegebenenfalls durch Halogen, Alkyl mit 1 bis 2 Kohlenstoffatomen und/oder Halogenalkyl mit 1 bis 2 Kohlenstoffatomen und 1 bis 5 gleichen oder verschiedenen Halogenatomen, wie Fluor- und Chloratomen, substituiertes Phenyl.

Die Isocyanate der Formel (VII) sind allgemein bekannte Verbindungen der organischen Chemie.

Die ferner als Ausgangsstoffe zu verwendenden Amine sind durch die Formel (VIII) allgemein definiert. In dieser Formel stehen $R^6$, $R^7$, $R^9$ und $R^{10}$ vorzugsweise für die Reste, die bei den Verbindungen der Formel (II) bereits vorzugsweise für diese Substituenten genannt wurden.

Die Amine der Formel (VIII) sind allgemein bekannte Verbindungen der organischen Chemie.

Die außerdem als Ausgangsstoffe zu verwendenden Alkohole sind durch die Formel (IX) allgemein definiert. In dieser

Formel steht $R^5$ vorzugsweise für die Reste, die bei den Verbindungen der Formel (II) bereits vorzugsweise für diesen Substituenten genannt wurden.

Die Alkohole der Formel (IX) sind allgemein bekannte Verbindungen der organischen Chemie.

Die weiterhin als Ausgangsstoffe zu verwendenden Alkylhalogenide der Formel (X) sind ebenfalls allgemein bekannte Verbindungen der organischen Chemie.

Als Nitrierungsmittel für das Verfahren (A) wird Nitriersäure (Salpetersäure-Schwefelsäure-Gemisch) eingesetzt.

Die Reaktionstemperaturen können bei der Durchführung des Verfahrens (A) in einem größeren Bereich variiert werden,. Im allgemeinen arbeitet man zwischen 0°C und 50°C, vorzugsweise zwischen 0°C und 30°C.

Die Durchführung des Verfahrens (A) und die Isolierung der Endprodukte erfolgen in üblicher Art und Weise.

Als Verdünnungsmittel kommen für das Verfahren (B) polare organische Lösungsmittel infrage. Hierzu gehören vorzugsweise Alkohole, wie Methanol oder Ethanol, sowie Nitrile, wie Acetonitril.

Die Umsetzung gemäß Verfahren (B) wird in Gegenwart eines Katalysators vorgenommen. Vorzugsweise werden Edelmetall-, Edelmetalloxid- bzw. Edelmetallhydroxid-Katalysatoren oder sogenannte 'Raney-Katalysatoren' verwendet, insbesondere Platin, Platinoxid und Nickel.

Die Reaktionstemperaturen können bei der Durchführung des Verfahrens (B) in einem größeren Bereich variiert werden. Im allgemeinen arbeitet man zwischen 20°C und 50°C, vorzugsweise bei 20°C bis 40°C.

Die Umsetzung gemäß Verfahren (B) kann bei Normaldruck, aber auch bei erhöhtem Druck, vorzugsweise bis 3 bar, durchgeführt werden.

Bei der Durchführung des Verfahrens (B) setzt man auf 1 Mol der Verbindung der Formel (IIa) etwa 1 Mol Wasserstoff und 0,1 Mol Katalysator ein. Zur Isolierung der Verbindungen der Formel (IIb) wird vom Katalysator abfiltriert, vom Lösungsmittel im Vakuum befreit und die erhaltenen Produkte werden durch Destillation bzw. Umkristallisation gereinigt.

Als Verdünnungsmittel kommen für das Verfahren (C) vorzugsweise inerte organische Lösungsmittel infrage. Hierzu gehören vorzugsweise Ketone, wie Aceton oder Methylethylketon; Nitrile, wie Acetonitril oder Propionitril; Ether, wie Tetrahydrofuran oder Dioxan; Formamide, wie Dimethylformamid; aromatische Kohlenwasserstoffe, wie Benzol oder Toluol; sowie halogenierte Kohlenwasserstoffe, wie Methylenchlorid oder Tetrachlorkohlenstoff.

Wird die Umsetzung gemäß Verfahren (C) in Gegenwart eines Säurebindemittels durchgeführt, so kann man alle üblicherweise verwendbaren anorganischen und organischen Säurebinder zugeben. Hierzu gehören vorzugsweise Kaliumcarbonat, Natriumcarbonat und Natriumhydrogencarbonat, ferner niedere tertiäre Alkylamine, Cycloalkylamine oder Arylaklylamine, wie z.B. Triethylamin, N,N-Dimethylbenzylamin, Dicyclohexylamin, sowie auch Pyridin und Diazabicyclooctan.

Die Reatkionstemperaturen können bei der Durchführung des Verfahrens (C/1.) in einem größeren Bereich variiert werden. Im allgemeinen arbeitet man zwischen 0°C und 100°C, vorzugsweise zwischen 10°C und 85°C.

Bei der Durchführung des Verfahrens (C/1.) setzt man vorzugsweise auf 1 Mol der Verbindung der Formel (IIb) 1 bis 2 Mol, für den Fall einer zweifachen Umsetzung 2 bis 4 Mol, an Halogen-Derivat der Formeln (IV), (V) oder (VI) und 1 bis 2 bzw. 2 bis 4 Mol Säurebinder ein. Die Isolierung der Endprodukte erfolgt in allgemein üblicher und bekannter Weise.

Die Reaktionstemperaturen können bei der Durchführung des Verfahrens (C/2.) in einem größeren Bereich variiert werden. Im allgemeinen arbeitet man zwischen 0°C und 100°C, vorzugsweise zwischen 20°C und 85°C.

Bei der Durchführung des Verfahrens (C/2.) setzt man vorzugsweise auf 1 Mol der Verbindung der Formel (IIb) 1 bis 2 Mol, für den Fall einer zweifachen Umsetzung 2 bis 4 Mol, an Isocyanat der Formel (VII) ein. Zur Isolierung der Endprodukte wird das Lösungsmittel abdestilliert und der Rückstand nach üblichen Methoden aufgearbeitet.

Die Reaktionstemperaturen können bei der Durchführung des Verfahrens (C/3.) in einem größeren Bereich variiert werden. Im allgemeinen arbeitet man zwischen -50°C und +100°C, vorzugsweise zwischen -50°C und +80°C.

Bei der Durchführung des Verfahrens (C/3.) setzt man vorzugsweise auf 1 Mol der Verbindung der Formel (IIb)1 bis 1,5 Mol, für den Fall einer zweifachen Umsetzung 2 bis 3 Mol, an Phosgen und 1 bis 1,5 Mol, für den Fall einer zweifachen Umsetzung 2 bis 3 Mol, an Amin der Formel (VIII) oder an Alkohol der Formel (IX) ein. Die Isolierung der Endprodukte erfolgt in allgemein üblicher und bekannter Weise.

Als Verdünnungsmittel kommen für das Verfahren (D) inerte organische Lösungsmittel infrage. Hierzu gehören vorzugsweise aromatische Kohlenwasserstoffe, wie Benzol, Toluol oder Xylol; Ester, wie Essigester; Ether, wie Tetrahydrofuran oder Dioxan; halogenierte Kohlenwasserstoffe, wie Methylenchlorid, Tetrachlorkohlenstoff oder Chlorbenzol; sowie Formamide, wie Dimethylformamid.

Die Umsetzung gemäß Verfahren (D) wird in Gegenwart einer Base durchgeführt. Hierbei können alle üblichen organischen und anorganischen Basen eingesetzt werden, wie beispielsweise Alkalimetallalkoholate, wie Natriummethylat oder Kalium-tert.-butylat; Alkalihydroxide und Alkalicarbonate, wie Natrium- und Kaliumhydroxid.

Die Reaktionstemperaturen können bei der Durchführung des Verfahrens (D) in einem größeren Bereich variiert werden. Im allgemeinen arbeitet man zwischen 0°C und 100°C, vorzugsweise zwischen 20°C und 100°C.

Bei der Durchführung des Verfahrens (D) setzt man vorzugsweise auf 1 Mol der Verbindung der Formel (IIc) 1 bis 1,2 Mol Alkylierungsmittel ein. Die Isolierung der Endprodukte erfolgt in allgemein üblicher und bekannter Weise.

Als Verdünnungsmittel kommen für das Verfahren (E) inerte organische Lösungsmittel infrage. Hierzu gehören vorzugsweise Alkohole, wie insbesondere Methanol. Die Umsetzung gemäß Verfahren (E) erfolgt durch Behandlung der Verbindungen der Formel (IId) mit wäßrig-alkalischer Lösung, wie vorzugsweise mittels wäßriger Natronlauge.
Die Umsetzung gemäß Verfahren (E) erfolgt vorzugsweise unter Rückflußtemperatur. Die Isolierung der Endprodukte erfolgt in allgemein üblicher und bekannter Weise.

Die Umsetzung gemäß Verfahren (F) erfolgt unter den oben genannten Bedingungen des Verfahrens (C).

Die Gewichtsverhältnisse der Wirkstoffe in den neuen Wirkstoffkombinationen können in relativ großen Bereichen schwanken. Im allgemeinen entfallen auf ein Gewichtsteil Photosynthesehemmer-Wirkstoff (herbizider Wirkstoff) 0,25 bis 100, vorzugsweise 5 bis 50, insbesondere 10 bis 20, Gew.-Teile 1,4-disubstituiertes Pyrazol-Derivat der Formel (II) (Synergist).

Die Photosynthesehemmer-Wirkstoffe weisen starke herbizide Wirkungen auf. Dennoch besitzen sie gegen einige Unkräuter, wie z. B. Galium aparine, Ipomoea hederacea, Datura stramonium, Cirsium arrense, Convolvulus arvensis oder Solanum nigrum und einige Ungräser, wie z. B. Agropyron repens, Avena fatua, Cynodon dactylon, Cyperus ssp. und Colium rigidum eine nicht immer ausreichende Wirkung. Die erfindungsgemäßen Wirkstoffkombinationen dehnen das Wirkungsspektrum der Verbindungen der Formeln (I-A bis I-J) aus und ermöglichen dadurch eine Bekämpfung dieser durch die herbiziden Wirkstoffe alleine nur schwer oder gar nicht bekämpfbaren Unkräuter.

Die erfindungsgemäßen Wirkstoffkombinationen können z.B. bei den folgenden Pflanzen verwendet werden:

Dikotyle Unkräuter der Gattungen: Sinapis, Lepidium, Galium, Stellarium, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffkombinationen ist jedoch keineswegs auf diese Gattungen beschränkt, sondern erstreckt sich in gleicher Weise auch auf andere Pflanzen.

Die erfindungsgemäßen Wirkstoffkombinationen zeigen insbesondere neben einer guten Wirkung gegen grasartige Unkräuter auch eine gute herbizide Wirkung bei breitblättrigen Unkräutern.

Die erfindungsgemäßen Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon,

Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anoranischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

In den Formulierungen können als weitere Zusätze Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffkombination, vorzugsweise zwischen 0,5 und 90 %.

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch als Einzelformulierungen bei der Anwendung gemischt, d.h. in Form von Tankmischungen zur Anwendung gebracht werden.

Die neuen Wirkstoffkombinationen können als solche oder in ihren Formulierungen weiterhin auch in Mischung mit anderen bekannten Herbiziden Verwendung finden, wobei wiederum Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die erfindungsgemäßen Wirkstoffkombinationen können sowohl vor als auch nach der Einsaat ebenso wie nach dem Auflaufen der Pflanzen gemeinsam oder in getrennten Anwendungen ausgebracht werden. Hierbei spielt die Reihenfolge der Anwendungen keine Rolle.

Beim Einsatz der erfindungsgemäßen Synergisten kann die übliche Aufwandmenge des Herbizids der Formeln (I-A bis I-J) verringert werden. Die Aufwandmenge an herbizidem Photosynthesehemmer-Wirkstoff liegt bei Flächenbehandlung zwischen 0,01 und 3,0 kg/ha, vorzugsweise zwischen 0,05 und 2,0 kg/ha.

Die Aufwandmenge an synergistischem 1,4-disubstituiertem Pyrazol-Derivat liegt bei Flächenbehandlung zwischen 0,1 und 10 kg/ha, vorzugsweise zwischen 0,5 und 3 kg/ha.

In entsprechenden Aufwandmengen zeigen die neuen Verbindungen der Formel (II) auch eine fungizide Wirkung, wie insbesondere gegen Getreidekrankheiten, Oomyceten, Apfelschorf und Pyricularia oryzae; weiterhin eine insektizide, einschließlich bodeninsektizide und wurzelsystemische sowie akarizide und nematizide Wirkung; ferner eine Wirkung gegen Hygiene- und Vorratsschädlinge.

Die gute herbizide Wirkung der erfindungsgemäßen Wirkstoffkombinationen geht aus den nachfolgenden Beispielen hervor. Während die einzelnen Wirkstoffe in der herbiziden Wirkung Schwächen aufweisen, zeigen die Kombinationen eine Unkrautwirkung, die über eine einfache Wirkungssummierung hinausgeht.

Ein synergistischer Effekt liegt bei Herbiziden immer dann vor, wenn die herbizide Wirkung der Wirkstoffkombination größer ist als die Summe der Wirkungen der applizierten Wirkstoffe.

Beispiel A

Pre-emergence-Test

Lösungsmittel: 5 Gew.-Teile Aceton
Emulgator: 1 Gew.-Teil Alkylarylpolyglykolether

Zur Herstellung einer zweckmäßigen Wirkstoffzubereitung vermischt man 1 Gew.-Teil herbiziden Wirkstoffs bzw. Synergisten bzw. eines Gemisches aus herbizidem Wirkstoff und Synergisten mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit einer Herbizid-Zubereitung bzw. mit der Synergisten-Zubereitung bzw. mit der Zubereitung aus Synergisten und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Wirkstoffe, Aufwandmenge und Resultate gehen aus der nachfolgenden Tabelle A hervor.

Tabelle A: Pre-emergence-Test

Synergistische Wirkung von 1,4-disubstituierten Pyrazol-Derivaten (II) (= Synergist S) und 4-Amino-6-tert.-butyl-3-methylthio-1,2,4-triazin-5-on (I-A-1) (=Herbizid H) an Ipomoea hederacea.

Die Aufwandmenge in kg/ha bezieht sich auf den Gehalt an Wirkstoff.

| Struktur des Synergisten | | (S) kg/ha | (H) kg/ha | % Schäden bei : Ipomoea hederacea H | S | H + S |
|---|---|---|---|---|---|---|
| 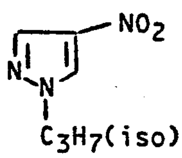 | (II-9) | 0,5 | 0,05 | 0 | 0 | 40 |
| | | 2,0 | 0,05 | 0 | 0 | 80 |
| | | 0,5 | 0,15 | 0 | 0 | 100 |
| | | 2,0 | 0,15 | 0 | 0 | 100 |
| 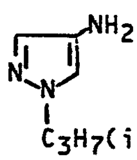 | (II-11) | 0,5 | 0,05 | 0 | 0 | 40 |
| | | 2,0 | 0,05 | 0 | 0 | 50 |
| | | 0,5 | 0,15 | 30 | 0 | 80 |
| | | 2,0 | 0,15 | 30 | 0 | 90 |
| 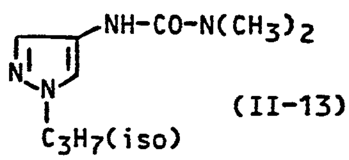 | (II-13) | 0,5 | 0,05 | 10 | 20 | 70 |
| | | 2,0 | 0,05 | 10 | 100 | 100 |
| | | 0,5 | 0,15 | 10 | 20 | 80 |
| | | 2,0 | 0,15 | 10 | 100 | 100 |
| 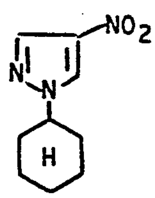 | (II-10) | 0,5 | 0,05 | 0 | 0 | 40 |
| | | 2,0 | 0,05 | 0 | 0 | 70 |
| | | 0,5 | 0,15 | 30 | 0 | 100 |
| | | 2,0 | 0,15 | 30 | 0 | 100 |

Tabelle A - Fortsetzung

| Struktur des Synergisten | (S) kg/ha | (H) kg/ha | % Schäden bei : Ipomoea hederacea H | S | H + S |
|---|---|---|---|---|---|
| 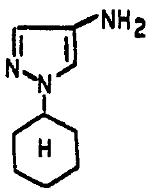 (II-12) | 0,5 | 0,05 | 0 | 0 | 10 |
| | 2,0 | 0,05 | 0 | 0 | 10 |
| | 0,5 | 0,15 | 30 | 0 | 30 |
| | 2,0 | 0,15 | 30 | 0 | 40 |
| 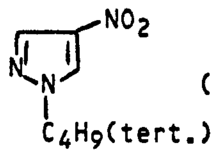 (II-1) | 0,5 | 0,05 | 0 | 0 | 20 |
| | 2,0 | 0,05 | 0 | 0 | 50 |
| | 0,5 | 0,15 | 10 | 0 | 50 |
| | 2,0 | 0,15 | 10 | 0 | 100 |
| 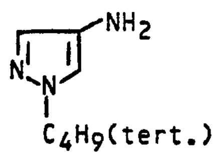 (II-2) | 0,5 | 0,05 | 0 | 0 | 10 |
| | 2,0 | 0,05 | 0 | 0 | 10 |
| | 0,5 | 0,15 | 10 | 0 | 20 |
| | 2,0 | 0,15 | 10 | 0 | 20 |
| 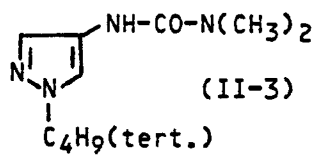 (II-3) | 0,5 | 0,05 | 0 | 10 | 10 |
| | 2,0 | 0,05 | 0 | 70 | 70 |
| | 0,5 | 0,15 | 10 | 10 | 50 |
| | 2,0 | 0,15 | 10 | 70 | 90 |
| 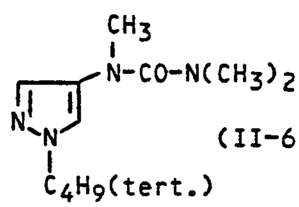 (II-6) | 0,5 | 0,05 | 0 | 0 | 40 |
| | 2,0 | 0,05 | 0 | 0 | 90 |
| | 0,5 | 0,15 | 10 | 0 | 90 |
| | 2,0 | 0,15 | 10 | 0 | 100 |
| 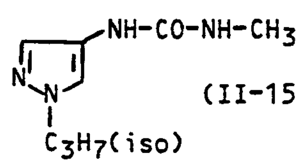 (II-15) | 0,5 | 0,05 | 30 | 10 | 90 |
| | 2,0 | 0,05 | 30 | 10 | 90 |
| | 0,5 | 0,15 | 30 | 20 | 50 |
| | 2,0 | 0,15 | 30 | 20 | 90 |
| 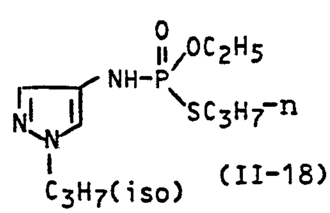 (II-18) | 0,5 | 0,05 | 30 | 10 | 30 |
| | 2,0 | 0,05 | 30 | 10 | 100 |
| | 0,5 | 0,15 | 30 | 10 | 50 |
| | 2,0 | 0,15 | 30 | 10 | 90 |

Tabelle A - Fortsetzung

| Struktur des Synergisten | kg/ha | kg/hg | % Schäden bei : Ipomoea hederacea H | S | H + S |
|---|---|---|---|---|---|
| 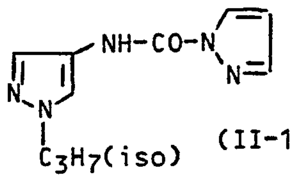 (II-19) | 0,5 | 0,05 | 30 | 20 | 50 |
| | 2,0 | 0,05 | 30 | 20 | 80 |
| | 0,5 | 0,15 | 30 | 20 | 50 |
| | 2,0 | 0,15 | 30 | 20 | 90 |
| 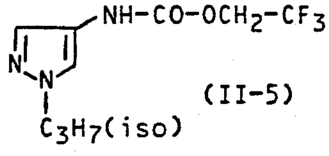 (II-5) | 0,5 | 0,05 | 30 | 10 | 60 |
| | 2,0 | 0,05 | 30 | 10 | 90 |
| | 0,5 | 0,15 | 30 | 10 | 100 |
| | 2,0 | 0,15 | 30 | 20 | 100 |
| 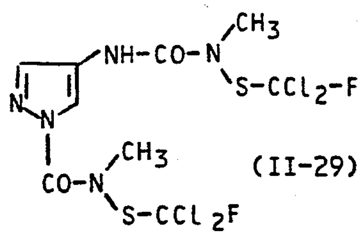 (II-29) | 0,5 | 0,05 | 30 | 20 | 30 |
| | 2,0 | 0,05 | 30 | 20 | 40 |
| | 0,5 | 0,15 | 30 | 20 | 70 |
| | 2,0 | 0,15 | 30 | 20 | 70 |
| 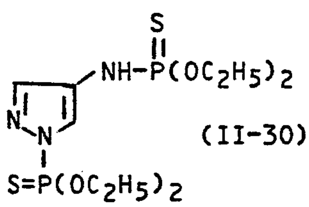 (II-30) | 0,5 | 0,05 | 30 | 10 | 90 |
| | 2,0 | 0,05 | 30 | 10 | 100 |
| | 0,5 | 0,15 | 30 | 10 | 100 |
| | 2,0 | 0,15 | 30 | 10 | 100 |
| 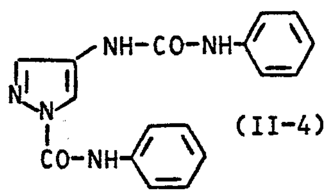 (II-4) | 0,5 | 0,05 | 30 | 10 | 10 |
| | 2,0 | 0,05 | 30 | 10 | 10 |
| | 0,5 | 0,15 | 30 | 10 | 50 |
| | 2,0 | 0,15 | 30 | 10 | 80 |
| 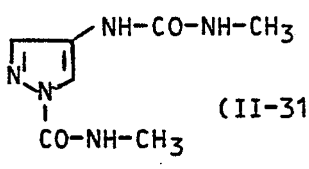 (II-31) | 0,5 | 0,05 | 30 | 10 | 0 |
| | 2,0 | 0,05 | 30 | 10 | 20 |
| | 0,5 | 0,15 | 30 | 10 | 20 |
| | 2,0 | 0,15 | 30 | 10 | 50 |

Tabelle A - Fortsetzung

| Struktur des Synergisten | (S) kg/ha | (H) kg/ha | % Schäden bei : Ipomoea hederacea H | S | H + S |
|---|---|---|---|---|---|
| 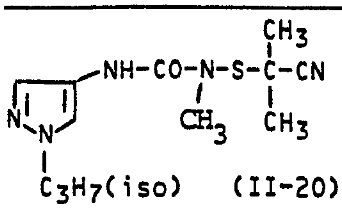 (II-20) | 0,5 | 0,05 | 30 | 0 | 20 |
| | 2,0 | 0,05 | 30 | 0 | 90 |
| | 0,5 | 0,15 | 30 | 0 | 90 |
| | 2,0 | 0,15 | 30 | 0 | 100 |
| 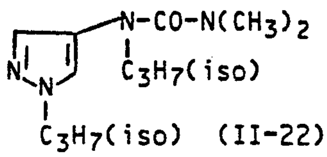 (II-22) | 0,5 | 0,05 | 30 | 10 | 90 |
| | 2,0 | 0,05 | 30 | 10 | 90 |
| | 0,5 | 0,15 | 30 | 10 | 90 |
| | 2,0 | 0,15 | 30 | 10 | 90 |
| 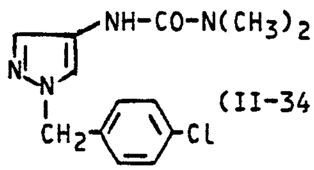 (II-34) | 0,5 | 0,05 | 30 | 10 | 50 |
| | 2,0 | 0,05 | 30 | 30 | 90 |
| | 0,5 | 0,15 | 30 | 10 | 90 |
| | 2,0 | 0,15 | 30 | 30 | 100 |
| 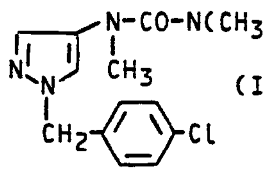 (II-35) | 0,5 | 0,05 | 30 | 0 | 20 |
| | 2,0 | 0,05 | 30 | 0 | 70 |
| | 0,5 | 0,15 | 30 | 0 | 50 |
| | 2,0 | 0,15 | 30 | 0 | 50 |
| 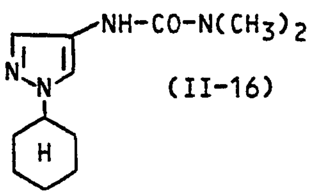 (II-16) | 0,5 | 0,05 | 30 | 10 | 0 |
| | 2,0 | 0,05 | 30 | 90 | 90 |
| | 0,5 | 0,15 | 30 | 10 | 90 |
| | 2,0 | 0,15 | 30 | 90 | 100 |
| 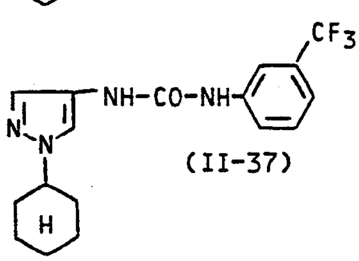 (II-37) | 0,5 | 0,05 | 30 | 0 | 10 |
| | 2,0 | 0,05 | 30 | 0 | 10 |
| | 0,5 | 0,15 | 30 | 0 | 100 |
| | 2,0 | 0,15 | 30 | 0 | 40 |

Tabelle A - Fortsetzung

| Struktur des Synergisten | (S) kg/ha | (H) kg/ha | % Schäden bei : Ipomoea hederacea H | S | H + S |
|---|---|---|---|---|---|
| 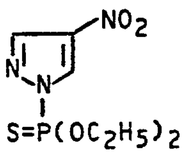 (II-38) | 0,5 | 0,05 | 30 | 0 | 0 |
| | 2,0 | 0,05 | 30 | 0 | 50 |
| | 0,5 | 0,15 | 30 | 0 | 100 |
| | 2,0 | 0,15 | 30 | 0 | 100 |
| 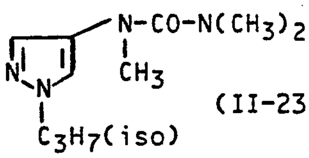 (II-23) | 0,5 | 0,05 | 30 | 10 | 50 |
| | 2,0 | 0,05 | 30 | 100 | 100 |
| | 0,5 | 0,15 | 30 | 10 | 100 |
| | 2,0 | 0,15 | 30 | 100 | 100 |
| 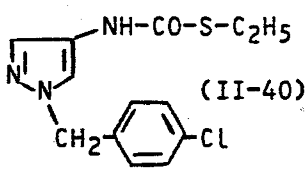 (II-40) | 0,5 | 0,05 | 30 | 0 | 10 |
| | 2,0 | 0,05 | 30 | 0 | 60 |
| | 0,5 | 0,15 | 30 | 0 | 80 |
| | 2,0 | 0,15 | 30 | 0 | 80 |
| 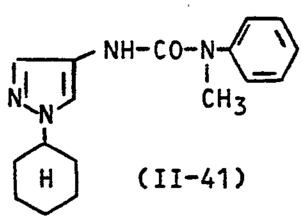 (II-41) | 0,5 | 0,05 | 30 | 0 | 20 |
| | 2,0 | 0,05 | 30 | 0 | 30 |
| | 0,5 | 0,15 | 30 | 0 | 50 |
| | 2,0 | 0,15 | 30 | 0 | 90 |

Die alleinige Anwendung des Herbizids (I-A-1) in einer Konzentration von 0,5 bzw. 2,0 kg/ha führt bei Ipomoea hederacea zu einer Schädigung von 80 bzw. 95 %.

Herstellungsbeispiele :

Beispiel 1 :

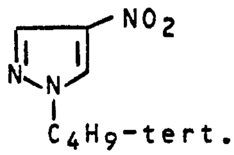

(II-1)

(Verfahren A)

Zu 75 ml konzentrierter Schwefelsäure tropft man unter Kühlung bei 20°C bis 30°C zunächst 37,2 g (0,3 Mol) 1-tert.-Butylpyrazol und anschließend bei 10°C bis 20°C langsam 93 ml 96-prozentige Salpetersäure (Dichte 1,5) zu. Man läßt das Reaktionsgemisch bei 20°C bis 30°C ausreagieren (ca. 2 Stunden), gießt es auf ca. 500 g Eis und saugt das Reaktionsprodukt ab. Man erhält 46 g (91 % der Theorie) 1-tert.-Butyl-4-nitropyrazol in Form gelber Kristalle vom Schmelzpunkt 70°C.

Herstellung des Ausgangsproduktes :

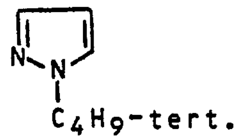

(III-1)

Zu einer Lösung von 62,3 g (0,5 Mol) tert-Butylhydrazin-Hydrochlorid in 100 ml Wasser gibt man bei 20°C 90 g (0,55 Mol) 1,1,3,3-Tetramethoxypropan; dann läßt man 75 ml konzentrierte Salzsäure zulaufen. Das Reaktionsgemisch erwärmt sich dabei auf ca. 50°C. Man läßt 18 Stunden ohne Kühlung ausreagieren, neutralisiert unter Kühlung bei 20°C bis 30°C durch Zugabe von konzentrierter wäßriger Natriumhydroxid-Lösung und schüttelt dann dreimal mit je 100 ml Methylenchlorid aus. Die organische Phase wird über Natriumsulfat getrocknet und bei 30°C/100 Torr eingedampft. Der Rückstand wird im Vakuum destilliert. Man erhält 56 g (90 % der Theorie) 1-tert.-Butylpyrazol in Form einer farblosen Flüssigkeit vom Siedepunkt 83°C bis 85°C/ 85 Torr.

Beispiel 2 :

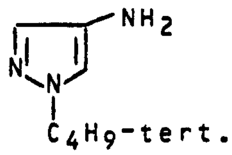

(II-2)

(Verfahren B)

Eine Lösung von 42,3 g (0,25 Mol) 1-tert.-Butyl-4-nitro-pyrazol (Beispiel II-1) in 250 ml Ethanol wird in Gegenwart von 5 g Raney-Nickel bei 50°C unter 55 bar Wasserstoffdruck hydriert. Dann filtriert man vom Katalysator, dampft das Filtrat im Vakuum ein und destilliert den Rückstand im Vakuum. Man erhält 27,8 g (80 % der Theorie) 1-tert.-Butyl-4-amino-pyrazol in Form eines erstarrenden gelbes Oels vom Siedepunkt 65°C/0,1 Torr und vom Schmelzpunkt 60°C.

Beispiel 3 :

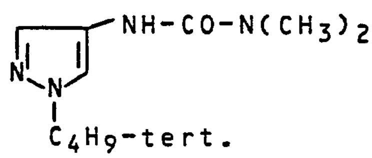

(II-3)

(Verfahren C/1.)

Eine Mischung von 16,7 g (0,12 Mol) 1-tert.-Butyl-4-amino-pyrazol (Beispiel II-2), 24,8 g (0,18 Mol) Kaliumcarbonat und 100 ml Acetonitril versetzt man ohne Kühlung mit 12,9 g (0,12 Mol) N,N-Dimethylcarbamoylchlorid und erwärmt dann 5 Stunden auf 60°C. Anschließend wird warm abgesaugt, mit warmem Acetonitril nachgewaschen und das Filtrat im Vakuum eingedampft. Man erhält 23 g (91 % der Theorie) N,N-Dimethyl-N'-(1-tert.-butyl-pyrazol-4-yl)-harnstoff in Form von rosa Kristallen vom Schmelzpunkt 64°C.

Beispiel 4 :

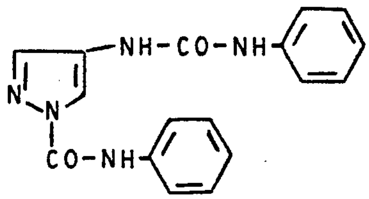

(II-4)

(Verfahren C/2.)

Zu einer Lösung von 8,9 g (0,1 Mol) 4-Aminopyrazol und 0,5 ml Trimethylamin in 200 ml Aceton tropft man bei 20°C bis 30°C 25 g (0,21 Mol) Phenylisocyanat. Das Gemisch wird 3 Stunden bei Raumtemperatur nachgerührt, dann saugt man das ausgefallene Produkt ab. Man erhält 25 g (78 % der Theorie) 1-Anilinocarbonyl-4-anilinocarbonylaminopyrazol in Form beiger Kristalle vom Schmelzpunkt 238°C.

Beispiel 5 :

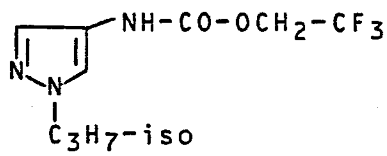

(II-5)

(Verfahren C/3.)

Zu einer Lösung von 7 g (0,07 Mol) Phosgen in 200 ml Toluol tropft man bei -50°C eine Mischung aus 7,2 g (0,071 Mol) Triethylamin, 8,8 g (0,07 Mol) 1-Isopropyl-4-aminopyrazol und 50 ml Toluol. Das Gemisch wird eine Stunde ohne Kühlung nachgerührt und dann bei 0°C bis 10°C mit einer Lösung von 7 g (0,07 Mol) 2,2,2-Trifluorethanol und 9,7 g (0,096 Mol) Triethylamin in 100 ml Toluol versetzt. Man rührt 3 Stunden bei Raumtemperatur nach, saugt vom ausgefallenen Salz ab und schüttelt das Filtrat zweimal mit je 100 ml Wasser. Dann wird die organische Phase über Natriumsulfat getrocknet und im Vakuum eingedampft. Man erhält 13 g (74 % der Theorie) N-(1-Isopropyl-pyrazol-4-yl)-carbamidsäure-2,2,2-trifluorethylester in Form eines Oels vom Brechungsindex $n_D^{22}$: 1,5066.

Beispiel 6 :

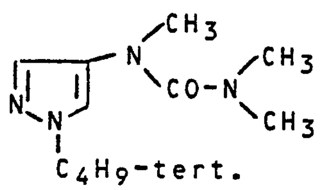

(II-6)

(Verfahren D)

Eine Lösung von 62 g (0,55 Mol) Kalium-tert.-butanolat in 500 ml Tetrahydrofuran wird mit 105 g (0,5 Mol) N,N-Dimethyl-N'-(1-tert.-butyl-pyrazol-4-yl)-harnstoff versetzt. Dazu tropft man 78 g (0,55 Mol) Methyljodid, wobei die Temperatur bis ca. 50°C ansteigt. Man läßt das Gemisch ohne Kühlung 18 Stunden ausreagieren, gibt 1 l Toluol zu und wäscht zweimal mit je 100 ml Wasser. Die organische Phase wird über Natriumsulfat getrocknet und im Vakuum eingedampft. Man erhält 93 g (82 % der Theorie) N,N,N'-Trimethyl-N'-(1-tert.-butyl-pyrazol-4-yl)-harnstoff in Form beiger Kristalle vom Schmelzpunkt 65°C.

Beispiel 7 :

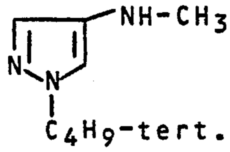

(II-7)

(Verfahren E)

Eine Mischung aus 500 ml Wasser, 200 ml konzentrierter wäßriger Natronlauge, 500 ml Methanol und 214 g (1,0 Mol) N-Methyl-N-(1-tert-butyl-pyrazol-4-yl)-carbamidsäure-methylester wird 4 Stunden unter Rückfluß erhitzt, dann abgekühlt, mit 1 l Wasser versetzt und zweimal mit je 300 ml Methylenchlorid extrahiert. Die organische Phase wird über Natriumsulfat getrocknet und im Vakuum eingedampft. Den Rückstand destilliert man im Vakuum. Man erhält 129 g (84 % der Theorie) 1-tert.-Butyl-4-methylamino-pyrazol in Form einer farblosen Flüssigkeit vom Siedepunkt 55°C/0,01 Torr.

Beispiel 8 :

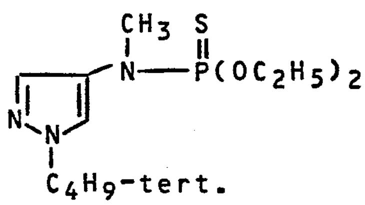

(II-8)

(Verfahren F)

Eine Mischung aus 10,8 g (0,07 Mol) 1-tert.-Butyl-4-methylamino-pyrazol (Beispiel II-7), 14,6 g (0,105 Mol) Kaliumcarbonat und 150 ml Acetonitril versetzt man bei 20°C bis 30°C mit 13,2 g (0,07 Mol) Thiophosphorsäure-O,O-diethylesterchlorid. Nach 4 Stunden gibt man 200 ml Toluol

zu und schüttelt zweimal mit je 50 ml Wasser aus. Die organische Phase wird über Natriumsulfat getrocknet und im Vakuum eingedampft. Man erhält 18,6 g (90 % der Theorie) Thiophosphorsäure-O,O-diethylester-N-methyl-N-(1-tert.-butyl-pyrazol-4-yl)-amid in Form eines gelben Oels vom Brechungsindex $n_D^{24}$: 1,5018.

In analoger Weise und entsprechend den angegebenen Verfahrensbedingungen können die folgenden Verbindungen der Tabelle 1 der allgemeinen Formel (II) erhalten werden :

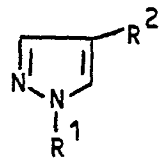

(II)

Tabelle 1 :

| Beisp. Nr. | $R^1$ | $R^2$ | FP.(°C) bzw. Kp.(°C)/Torr bzw. Brechungsindex |
|---|---|---|---|
| II-9 | i-$C_3H_7$ | $-NO_2$ | 63 |
| II-10 | 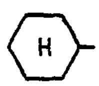 | $-NO_2$ | 65 |
| II-11 | i-$C_3H_7$ | $-NH_2$ | 78/0,1 |
| II-12 | 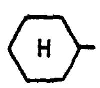 | $-NH_2$ | 39 |
| II-13 | i-$C_3H_7$ | $-NH-CO-N(CH_3)_2$ | 106 |
| II-14 | i-$C_3H_7$ | $-NH-COOCH_3$ | $n_D^{20}$: 1,5022 |

Tabelle 1 - Fortsetzung

| Beisp.-Nr. | $R^1$ | $R^2$ | Fp.(°C) bzw. Kp.(°C)/Torr bzw. Brechungsindex |
|---|---|---|---|
| II-15 | $i-C_3H_7$ | $-NH-CO-NHCH_3$ | 112 |
| II-16 | 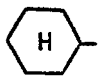 | $-NH-CO-N(CH_3)_2$ | 150 |
| II-17 | $tert.-C_4H_9$ | $-NH-COOCH_3$ | $n_D^{22}$: 1,4973 |
| II-18 | $i-C_3H_7$ | 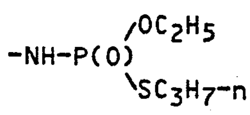 | $n_D^{22}$: 1,5097 |
| II-19 | $i-C_3H_7$ | 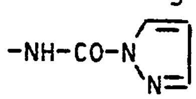 | $n_D^{22}$: 1,5703 |
| II-20 | $i-C_3H_7$ | $-NH-CO-N(CH_3)-S-C(CH_3)_2-CN$ | 94 |
| II-21 | $i-C_3H_7$ | $-NH-P(S)(OC_2H_5)_2$ | $n_D^{19}$: 1,5149 |
| II-22 | $i-C_3H_7$ | $-N(C_3H_7-i)-CO-N(CH_3)_2$ | $n_D^{26}$: 1,4962 |
| II-23 | $i-C_3H_7$ | $-N(CH_3)-CO-N(CH_3)_2$ | $n_D^{21}$: 1,5063 |
| II-24 | $tert.-C_4H_9$ | $-N(CH_3)-COOCH_3$ | $n_D^{22}$: 1,4930 |
| II-25 | 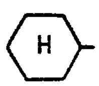 | $-N(CH_3)-CO-N(CH_3)_2$ | $n_D^{21}$: 1,5260 |
| II-27 | $tert.-C_4H_9$ | $-N(CH_3)-CO-N(CH_3)-SCCl_2F$ | $n_D^{24}$: 1,5181 |
| II-28 | $tert.-C_4H_9$ | $-N(CH_3)-CO-NHCH_3$ | 100 |

Tabelle 1 - Fortsetzung

| Beisp. Nr. | $R^1$ | $R^2$ | Fp.(°C) bzw. Kp.(°C)/Torr bzw. Brechungsindex |
|---|---|---|---|
| II-29 | $-CO-N(CH_3)-SCCl_2F$ | $-NH-CO-N(CH_3)-SCCl_2F$ | 1,5788 |
| II-30 | $-P(S)(OC_2H_5)_2$ | $-NH-P(S)(OC_2H_5)_2$ | $n_D^{20}$: 1,5240 |
| II-31 | $-CO-NHCH_3$ | $-NH-CO-NHCH_3$ | 210 |
| II-32 | $-COOCH_2CCl_3$ | $-NH-COOCH_2CCl_3$ | 154 |
| II-33 | $-CH_2-$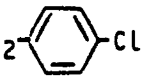 | $-NH_2$ | 55 |
| II-34 | $-CH_2-$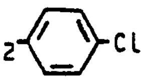 | $-NH-CO-N(CH_3)_2$ | 151 |
| II-35 | $-CH_2-$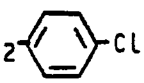 | $-N(CH_3)-CO-N(CH_3)_2$ | 106 |
| II-36 | 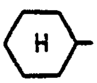 | -NH-CO-NH-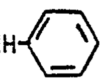 | 216 |
| II-37 | 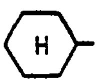 | -NH-CO-NH-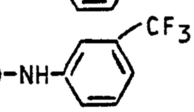 | 143 |
| II-38 | $-P(S)(OC_2H_5)_2$ | $-NO_2$ | $n_D^{21}$: 1,5242 |
| II-39 | $-CO-NHCH_3$ | $-NO_2$ | 190 |
| II-40 | $-CH_2-$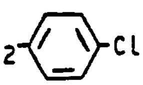 | $-NH-COSC_2H_5$ | 131 |
| II-41 | 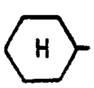 | $-NH-CO-N(CH_3)-$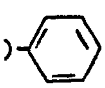 | 142 |
| II-42 | sec.-$C_4H_9$ | $-NO_2$ | |

Tabelle 1 - Fortsetzung

| Beisp. Nr. | $R^1$ | $R^2$ |
|---|---|---|
| II-43 | $C_2H_5-C(CH_3)_2$ | $-NO_2$ |
| II-44 | sec.-$C_4H_9$ | $-NH_2$ |
| II-45 | $C_2H_5-C(CH_3)_2-$ | $-NH_2$ |
| II-46 | tert.-$C_4H_9$ | $-NH-COCH_3$ |
| II-47 | sec.-$C_4H_9$ | $-NH-CO-N(CH_3)_2$ |
| II-48 | $C_2H_5-C(CH_3)_2$ | $-NH-CO-N(CH_3)_2$ |
| II-49 | tert.-$C_4H_9$ | $/OC_2H_5$<br>$-NH-P(O)$<br>$\backslash SC_3H_7-n$ |
| II-50 | tert.-$C_4H_9$ | -NH-CO-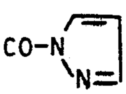 |
| II-51 | tert.-$C_4H_9$ | $-NH-CO-N(CH_3)-S-C(CH_3)_2-CN$ |
| II-52 | tert.-$C_4H_9$ | $-NH-P(S)(OC_2H_5)_2$ |
| II-53 | sec. $C_4H_9$ | $-N(CH_3)-CO-N(CH_3)_2$ |
| II-54 | $C_2H_5-C(CH_3)_2$ | $-N(CH_3)-CO-N(CH_3)_2$ |
| II-55 | tert.-$C_4H_9$ | $-N(C_3H_7-i)-CO-N(CH_3)_2$ |

Tabelle 1 - Fortsetzung

| Beisp. Nr. | $R^1$ | $R^2$ |
|---|---|---|
| II-56 | $i-C_3H_7-$ | $-N(CH_3)-COOCH_3$ |
| II-57 | $i-C_3H_7-$ | $-N(CH_3)-COCH_3$ |
| II-58 | $tert.-C_4H_9-$ | $-N(CH_3)-COCH_3$ |
| II-59 | $tert.-C_4H_9-$ | $-NH-C_2H_5$ |
| II-60 | $i-C_3H_7-$ | $-NH-CH_3$ |
| II-61 | 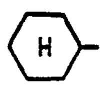 | $-NH-CH_3$ |

Entsprechend Beispiel 1 und den angegebenen Verfahrensbedingungen können die folgenden Ausgangsprodukte der Tabelle 2 der allgemeinen Formel (III) erhalten werden :

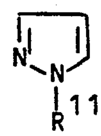

(III)

Tabelle 2 :

| Beisp.-Nr. | $R^{11}$ | Physikalische Konstante |
|---|---|---|
| III-2 | $i-C_3H_7-$ | Kp.: 38°C/9 Torr |
| III-3 | 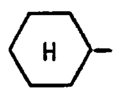 | Kp.: 105°C/15 Torr |
| III-4 | $sec.-C_4H_9-$ | |
| III-5 | $C_2H_5-C(CH_3)_2-$ | |

Patentansprüche

1. Herbizide Mittel, gekennzeichnet durch einen Gehalt an einer Wirkstoffkombination, bestehend aus

(a) einem Photosynthesehemmer-Wirkstoff (Herbizid) und

(b) einem 1,4-disubstituierten Pyrazol-Derivat der allgemeinen Formel (II) (Synergist),

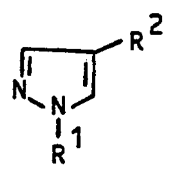

(II)

in welcher

$R^1$ für Alkyl mit mehr als zwei Kohlenstoffatomen, gegebenenfalls substituiertes Cycloalkyl, substituiertes Benzyl, sowie die Gruppierungen

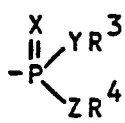 ; $-CO-XR^5$ und 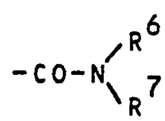 steht,

$R^2$ für Amino, Alkylamino sowie die Gruppierungen

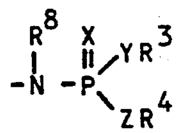 , 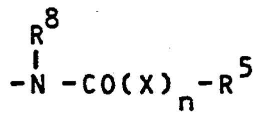 und 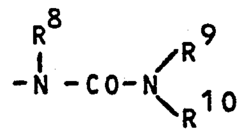 steht,

sowie auch für Nitro steht, wenn $R^1$ nicht für substituiertes Benzyl steht;

$R^3$ für Alkyl steht;

$R^4$ für Alkyl steht;

$R^5$ für Alkyl oder Halogenalkyl steht;

$R^6$ für Wasserstoff oder Alkyl steht;

$R^7$ für Alkyl, Halogenalkylthio, Cyanalkylthio oder gegebenenfalls substituiertes Phenyl steht, wobei jedoch $R^6$ und $R^7$ nicht gleichzeitig für Methyl stehen;

$R^8$ für Wasserstoff oder Alkyl steht;

$R^9$ für Wasserstoff oder Alkyl steht;

$R^{10}$ für Alkyl, Halogenalkylthio, Cyanalkylthio oder gegebenenfalls substituiertes Phenyl steht;

$R^9$ und $R^{10}$ gemeinsam mit dem Stickstoffatom, an das sie gebunden sind, für einen Heterocyclus stehen, der weitere Heteroatome enthalten kann;

X, Y und Z für Sauerstoff oder Schwefel stehen und der Index

n für 0 oder 1 steht.

2. Herbizide Mittel gemäß Anspruch 1, dadurch gekennzeichnet, daß sie als Photosynthesehemmer-Wirkstoffe (Herbizide) Verbindungen aus den nachfolgend genannten Wirkstoffgruppen der Formeln (I-A) bis (I-J) enthalten:

(A) Triazinon-Derivate der Formel

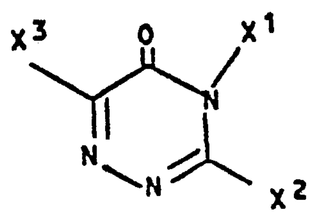

(I-A)

in welcher

$X^1$ für Amino, gegebenenfalls substituiertes Alkylidenamino oder Alkyl mit 1 bis 2 Kohlenstoffatomen steht;

$X^2$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^3$ für gegebenenfalls durch Halogen substituiertes tert.-Butyl oder gegebenenfalls substituiertes Phenyl steht;

(B) Triazindion-Derivate der Formel

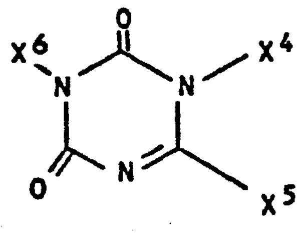

(I-B)

in welcher

$X^4$ für Amino, gegebenenfalls substituiertes Alkylidenamino oder Alkyl mit 1 bis 2 Kohlenstoffatomen steht;

$X^5$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^6$ für Alkyl mit 1 bis 6 Kohlenstoffatomen oder gegebenenfalls substituiertes Phenyl steht;

(C) Triazin-Derivate der Formel

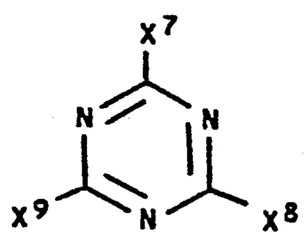

(I-C)

in welcher

$X^7$ für Chlor, Alkoxy oder Alkylthio mit jeweils 1 bis 2 Kohlenstoffatomen steht;

$X^8$ für Alkylamino mit 1 bis 4 Kohlenstoffatomen im Alkylteil steht; und

$X^9$ für gegebenenfalls durch Cyano substituiertes Alkyl mit 1 bis 4 Kohlenstoffatomen steht;

(D) Harnstoff-Derivate der Formel

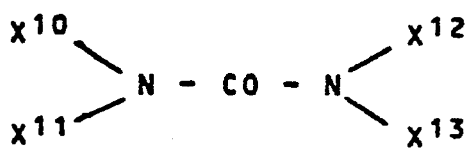

(I-D)

in welcher

$X^{10}$ für gegebenenfalls substituiertes Phenyl, Benz-thiazolyl oder gegebenenfalls substituiertes Thiadiazolyl steht;

$X^{11}$ für Wasserstoff oder Methyl steht;

$X^{12}$ für Methyl steht; und

$X^{13}$ für Wasserstoff, Alkyl mit 1 bis 4 Kohlenstoffatomen, Alkoxy mit 1 bis 2 Kohlenstoffatomen oder Alkinyl mit 2 bis 4 Kohlenstoffatomen steht;

(E) Carboxanilid-Derivate der Formel

$$X^{14} - CO - NH - X^{15} \qquad (I-E)$$

in welcher

$X^{14}$ für Alkyl mit bis zu 6 Kohlenstoffatomen, Alkoxy mit 1 bis 4 Kohlenstoffatomen, Alkenyl mit 2 bis 4 Kohlenstoffatomen oder Cycloalkyl mit 3 bis 6 Kohlenstoffatomen steht; und

$X^{15}$ für gegebenenfalls substituiertes Phenyl steht;

(F) Uracil-Derivate der Formel

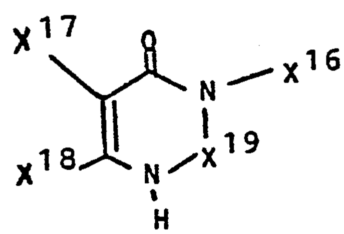

(I-F)

in welcher

$X^{16}$ für Alkyl mit 1 bis 6 Kohlenstoffatomen oder Cycloalkyl mit 5 bis 7 Kohlenstoffatomen steht;

$X^{17}$ für Halogen steht;

$X^{18}$ für Alkyl mit 1 bis 2 Kohlenstoffatomen steht, oder

$X^{17}$ und $X^{18}$ gemeinsam für eine gegebenenfalls substituierte Alkylenkette oder einen gegebenenfalls substituierten anellierten Benzolring stehen; und

$X^{19}$ für die -CO- oder $-SO_2$-Gruppe steht;

(G) Biscarbamat-Derivate der Formel

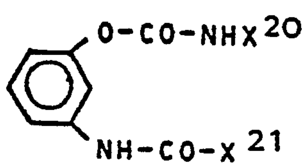

(I-G)

in welcher

$X^{20}$ für Alkyl mit 1 bis 4 Kohlenstoffatomen oder gegebenenfalls substituiertes Phenyl steht; und

$X^{21}$ für Alkoxy mit 1 bis 4 Kohlenstoffatomen oder Dialkylamino mit 1 bis 2 Kohlenstoffatomen in jedem Alkylteil steht;

(H) Pyridazinon-Derivate der Formel

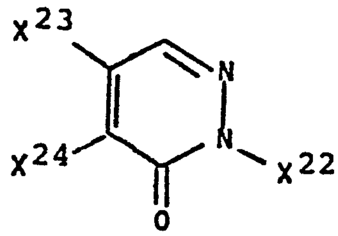

(I-H)

in welcher

$X^{22}$ für gegebenenfalls substituiertes Phenyl steht;

$X^{23}$ für Amino, Alkylamino oder Dialkylamino mit jeweils 1 oder 2 Kohlenstoffatomen in jedem Alkylteil steht; und

$X^{24}$ für Halogen steht;

(J) Hydroxybenzonitril-Derivate der Formel

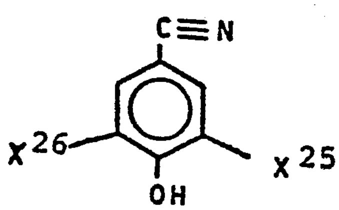

(I-J)

in welcher

$X^{25}$ für Halogen steht; und

$X^{26}$ für Halogen steht.

3. Herbizide Mittel gemäß Anspruch 1, dadurch gekennzeichnet, daß sie als Photosynthesehemmer-Wirkstoff (Herbizid) 4-Amino-6-tert.-butyl-3-methylthio-1,2,4-triazin-5-on der Formel (I-A-1) (Metribuzin)

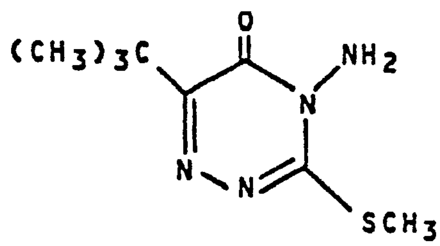

enthalten.

4. Herbizide Mittel gemäß Anspruch 1, dadurch gekennzeichnet, daß in der Wirkstoffkombination das Gewichtsverhältnis von (1) Photosynthesehemmer-Wirkstoff (Herbizid) zu (2) dem 1,4-disubsituierten Pyrazol-Derivat der Formel (II) (Synergist) zwischen 1 : 0,25 und 1 : 100 liegt.

5. Herbizide Mittel gemäß Anspruch 4, dadurch gekennzeichnet, daß das angegebene Gewichtsverhältnis zwischen 1 : 5 und 1 : 50 liegt.

6. Herbizide Mittel gemäß Anspruch 5, dadurch gekennzeichnet, daß das angegebene Gewichtsverhältnis zwischen 1 : 10 und 1 : 20 liegt.

7. Verwendung von Wirkstoffkombinationen gemäß Ansprüchen 1 bis 6 zur Bekämpfung von Unkraut.

8. Verfahren zur Herstellung von herbiziden Mitteln, dadurch gekennzeichnet, daß man eine Wirkstoffkombination gemäß Ansprüchen 1 bis 6 mit Streckmitteln und/oder oberflächenaktiven Mitteln vermischt.

9. Verwendung von 1,4-disubstituierten Pyrazol-Derivaten der Formel (II) gemäß Anspruch 1 als Synergisten in Kombination mit Photosynthesehemmer-Herbiziden.

10. Verwendung von 1,4-disubstituierten Pyrazol-Derivaten der Formel (II) gemäß Anspruch 1 als Synergisten in Kombination mit dem herbiziden Wirkstoff 4-Amino-6-tert.-butyl-3-methylthio-1,2,4-triazin-5-on (Metribuzin) der Formel (I-A-1) gemäß Anspruch 3.

11. 1,4-Disubstituierte Pyrazol-Derivate der allgemeinen Formel (II)

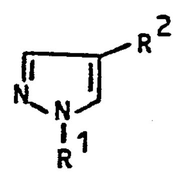

(II)

in welcher $R^1$ und $R^2$ die in Anspruch 1 angegebene Bedeutung haben.